# EUROPEAN PATENT APPLICATION

(11) **EP 1 348 961 A1**
(43) Date of publication of application: **01.10.2003**
(21) Application number: 01982082.8
(22) Date of filing: 30.08.2001
(51) Int. Cl.: G01N 33/543, G01N 33/574, G01N 33/68

(54) **PROTEIN CHIPS, METHOD PRODUCING IT AND DETECTION SYSTEM OF THE PROTEIN CHIPS, AND OPERATING METHOD OF THE DETECTION SYSTEM**

(30) Priority: 04.01.2001 CN 01105023
(71) Applicant: Shanghai Health Digit Limited, 200233 Shanghai (CN)
(72) Inventor: HU, Gengxi, Shanghai 200233 (CN)
(74) Representative: Geissler, Bernhard, Dr. jur., Dipl.-Phys.
(86) International application number: CN0101294
(87) International publication number: WO02054070

(57) **Abstract**

The invention is related to protein chips, the methods producing it, the use thereof, wherein the protein chip can operate multiple index detection in parallel. The protein chip comprises a solid carrier and proteins being detectable multiple index in parallel, the various of proteins are fixed on the carrier in order by automatic sample system. The parts that does not drop samples on the chip are blocked by blocking solution, lyophilized and stored. The invention is also related to the detection system of the protein chip above, comprising protein chip, mixture of various labelled proteins with peroxidase, diluent, chemical composition that can perform chemoluminescence reaction, detergent and standard. The principle is: contacting the solid carrier combined with various proteins A that can be specially binding substance in vivo with sample solutions containing target proteins B, forming stable complex A-B, further reaction with the corresponding labelled AC-labelled specially binding protein to form stable A-B-C- labelled, in which the label is a chemoluminescence marker that can give detectable signal. The protein chip can significantly improve detection accuracy, and can advantageously give high reliability and detection sensibility. The protein chips are broadly useful in areas of molecular biology, bio-medicine, human proteinic study, especially clinical diagnosis.

## Description

### FIELD OF INVENTION

The invention is related to a protein chip, the methods producing it, the use thereof, wherein the protein chip can accomplish parallel detection of multiple markers.

### BACKGROUND OF INVENTION

Biochip technology, a novel biotechnology sprung up in the mid-1990s, has been used in many biological fields. Researchers have attached great importance to this technology since it can simultaneously analyze information of many bio-molecules in one reaction.

Based on different test object, biochip is mainly divided into Gene chip and Protein chip. Since gene chip plays an important role in the research of Human Genome Project (HGP), the development of this technology has got great progresses. With the accomplishment of HGP, many top laboratories and biological scientists have changed the emphasis of their research to proteomics, which can be used to interpret the function of those known genes.

At present, cDNA array technology is often used to study various proteins simultaneously. However it has many limitations since the interactions of proteins, which mainly include antigen-antibody interactions and receptor-ligand interactions, are based on complementation of protein conformation, and not determined by the specificity of their DNA sequences.

Protein Chip technology, however, is a better way to study the function of proteins. Using the microarray composed by many proteins immobilized on a solid substrate, Protein Chip technology can detect directly how a protein of certain function works. But the development and application of this technology is still limited for its great research difficulties.

Most methods now used to detect urines, sera and other body fluids, such as ELISA, radioactive isotope assay, colloidal gold-labeled assay, fluorescence assay, chemiluminescence assay, time resolved fluorescence assay etc, can only detect single marker and get little information. In addition, they have other limitations such as complicated procedure, expensive apparatus and reagents, tiring operation, low sensitivity and easy contamination.

### SUMMARY OF INVENTION

The present invention provides a protein chip that can accomplish parallel detection of multiple markers.

The present invention also provides a method for preparing the above protein chip.

The present invention also provides a detection system mainly composed by the above protein chip.

The present invention also provides the method about how to use the above detection system.

According to the present invention, the preparation of said protein chip for parallel detection of multiple markers is based on the principles as follows:
1) Since proteins can bind with the solid substrate by physical absorption or covalent linkage, various proteins can be immobilized on a solid substrate in high density by an automatic spotting system;
2) Since regions without immobilized proteins on the solid substrate can randomly absorb proteins and other small molecules, which will affect the accuracy of protein chip, these regions should be blocked with a blocking solution after the proteins are immobilized on the substrate;
3) In order to keep the activity of proteins for a long time, the blocked solid substrate need to be stored at low temperature.

According to the present invention, the preparation method of said protein chip for parallel detection of multiple markers comprises the steps of:
1) respectively dissolving each protein to be immobilized to a certain concentration with coating solution;
2) immobilizing all the proteins on a solid substrate by an automatic spotting system in a density of 25-200 dot/cm², wherein the amount of the said protein ranges from 0.1 to 10ng/dot;
3) leaving the protein chip overnight;
1) blocking the protein chip with blocking solution; drying and storing at 4□.

The said protein chip in the present invention for parallel detection of multiple markers has following characters:
1) Said solid substrate can be inorganic substrate or organic substrate. Inorganic substrate can be semiconductor silicon substrate, glass substrate, micro-pore silicon substrate, micro-pore glass etc, among which the glass substrate is preferred. Organic substrate can be cellulose acetate membrane, nitrocellulose membrane, nylon membrane, polypropylene membrane and so on, among which the nitrocellulose membrane is preferred.
2) Said proteins can be antigens, antibodies, receptors, ligands and so on, furthermore, they can be proteins that can specifically bind with disease-related proteins, especially referring to tumor markers. Said receptors can be biomolecules on cell membrane or in cells, which can recognize and bind with bioactive molecules (such as drugs, toxins, transmitters, antigens, cell adhesion molecules and so on). Receptors can transfer the information produced by biomolecules to effectors, and lead to corresponding bioeffects. Most of biomolecules are proteins, and very few are glycolipids. Said ligands are bioactive molecules (such as drugs, toxins, transmitters, antigens, cell adhesion molecules and so on), which can specifically bind with receptors. The receptors and ligands said in the present invention are proteins.
3) The concentrations of proteins on the chip are pre-adjusted so that the intensity of different chemiluminescent signals generated after a series of protein reactions could be in the range of two adjacent orders of magnitude (intensity of the strongest signal is no more than 100 folds of that of the weakest signal). Thus it is possible to use the same apparatus to fulfill the measurement..
4) The coating solution is CBS (NaHCO₃-Na₂CO₃) at pH9.0-10.0 (9.6 is the optimum pH).
   The coating solution is used to provide a pH range so that immobilized proteins could combine stronger with the solid substrate by covalent linkage or physical absorption.
5) The blocking solution is TBS (0.7-0.9%NaCl, 1.0-1.5%Tris) containing 0.1-0.5%Tween20, 0.02-0.3%tyrosine, 1-9%sucrose, 1-9%BSA and 0.1-1%proclin. The optimum ingredient is TBS containing 0.2%Tween20, 0.1%tyrosine, 4%sucrose, 5%BSA and 0.5%proclin. The blocking solution has the following advantages: both tyrosine and BSA have blocking function; proclin is antiseptic; sucrose is an inert substance and can isolate air; BSA and sucrose can support the frame structure. The blocking solution prevents the proteins from combine with other regions of the solid substrate, and therefore ensures the accuracy of data.
   Other blocking solutions have also been tried, such as TTBS buffer (TBS containing 0.1%(v/v) Tween); TBS containing 10% skim milk; TTBS containing 0.2% tyrosine;(Related references: *Bejurrum and Schater-Nielsen, 1986; Gillespie and Hudspeth, 1991; Harlow and Lane, 1988; Schneppenheim et al, 1991*) and PBS containing 5% (v/v) skim milk. (Related reference: *Molecular Cloning: A Laboratory Manual (2nd ed): J. sambrook, E.F.Fritsch T. Maniatis*)
   The analysis of signal-to-noise ratio using different blocking solutions showed that the blocking solution provided in the present invention is much better than any others mentioned above.
6) The samples to be detected in present invention include various body fluids, which are convenient to collect and harmless to patients.

The unit of concentration mentioned above is W/V, without specific definition.

The principle of the protein chip in present invention is: various proteins immobilized on protein chip (hereinafter referred to as "B") can specifically combine with corresponding proteins in sample body fluids, tissues, or cells and so on (hereinafter referred to as "A"). The formed protein complexes (B-A) can further combine with another special binding proteins (hereinafter referred to as "C") that is corresponding to A to form stable B-A-C-labeled, wherein C have been labeled with peroxidase (hereinafter referred to as "C-labeled"). Since the peroxidase can catalyze a chemiluminescence reaction and generate light signal, special biochip reader is used to quantitatively determine the intensity of generated light signals. Therefore, determining the intensity of light signals can calculate the amount of proteins (A) in sample.

According to the present invention, the protein chip detection system of for parallel detection of multiple markers comprises:
(a) a protein chip for parallel detection of multiple markers;
(b) a mixture of various proteins labeled with peroxidase;
(c) a diluent for diluting sample body fluids;
(d) a chemical composition that can generate and enhance chemiluminescence reaction;
(e) a washing solution;
(f) a series of standards of proteins to be detected.

According to present invention, the detecting method of using the protein chip detection system for parallel detection of multiple markers comprises following steps:
1) diluting the supernatant liquid of sample 1:5-20 with the diluent;
2) sucking the supernatant liquid of sample and dropping them onto the surface of protein chip with a density of 500µl volume per cm²; vibrating at a frequency of 200-300 r/min and a temperature of 20-37□ for 20-30 minutes; proteins immobilized on protein chip (B) can specifically react with the corresponding proteins in the sample(A), and forming stable complex B-A;
3) removing the solution after the reaction; immersing the protein chip in washing solution at 20-37□ and vibrating for 3-6 minutes; repeating for several times;
4) sucking the mixture of various proteins labeled with peroxidase (C-labeled) and dropping it onto the surface of protein chip with a density of 500µl volume per cm²; vibrating at a frequency of 200-300 r/min and a temperature of 20-37□ for 20-30 minutes, so that B of complex (B-A) can combine with C-labeled to form stable A-B-C-labeled complex;
5) removing the solution after the reaction; immersing the protein chip in washing solution at 20-37□ and vibrating for 3-6 minutes; repeating for several times;
6) drying the chip at room temperature; dropping the chemical composition which can generate chemiluminescence onto chip surface with a density of 100µl volume per cm²; reacting for 3-5 minutes at 20-37□;
7) putting the protein chip into biochip reader and measuring the light signals generated on the protein chip.

The detection system of protein chip for parallel detection of multiple markers has following characters: wherein the unit of concentration is WN, without specific definition.
1) The mixture of various proteins is the mixture of various proteins labeled with peroxidase, in which proteins can specifically bind with target proteins in sample body fluids.
2) The label is peroxidase, especially referred to horseradish peroxidase.
3) The washing solution comprises 0.7-0:9%NaCl, 1.0-1.5%Tris, 0.05-0.2%Tween 20 and pH ranges from 6.0 to 9.0. The optimum ingredient of washing solution comprises 0.9%NaCI, 1.21%Tris, and 0.1%Tween20 at pH 7.50.
4) The diluent comprises 0.7-0.9%NaCI, 1.0-1.5%Tris, 0.05-0.2%Tween20 and 0.05-0.2%tyrosine. The optimum ingredient of diluent comprises 0.9%NaCl, 1.21%Tris, 0.1%Tween20 and 0.1%tyrosine.
5) The ingredient of stable solution is: 70-80% A solution 5-20%(VN) +glycol (VN) + 1-9% sucrose (W/V) +0.1-1% proclin (VN) +0.05-0.5%EDTA 2Na (A solution comprises 40-60% 0.05M PBS (pH7.2) and 40-60% Fetal Bovine Serum). The optimum ingredient is: 80% A solution (VN) + 19% glycol (VN) + 3% sucrose (WN) +0.5% proclin (VN) +0.2%EDTA·2Na (A solution comprises 60% 0.05M PBS (pH7.2) and 40% Fetal Bovine Serum).
6) Serial standards are the mixture of various proteins for detection in different concentration, which can specifically bind with the proteins immobilized on protein chip. After the reaction, light signals generated on the chip can be detected with a biochip reader. A protein marker in the sample would be defined as positive marker if the corresponding signal produced after protein chip reacting with sample, remarkably higher than that reacting with the standards controls. The standards are redissolved with deionized water before used.
7) There are two kinds of chemiluminescence reactions: Chemiluminescence and Enhanced Chemiluminescence. The chemical composition to generate chemiluminescence using in the present invention is based on enhanced chemiluminescence. The reagents involving in the reaction are Luminol (5-amino-2, 3-dihydro-1, 4-phthalazinedione Sodium Salt), H₂O₂, Enhancer (Eosin or PIP) and peroxidase (such as horseradish peroxidase, HRP), which are all purchased from Sigma.

According to present invention, one of the remarkable advantages of the protein chip for parallel detection of multiple markers is that it can be used to simultaneously detect multiple disease-related markers, thus increasing the detection accuracy greatly. Most of present disease-related markers now used are non-specific markers. The amount of multiple markers will simultaneously increase in one disease, and the amount of the same marker will increase in multiple diseases. This leads to the difficulty of disease diagnosis and even causes misdiagnosis.

All tumor markers being widely used nowadays are not specific markers. The positive ratio of AFP (α-Fetoprotein) to detect hepatocellular carcinoma is only 70%, which is known as the best tumor marker. And AFP is also increased in other diseases such as testicular cancer, freaky cancer, gastric cancer and pancreatic cancer. Thus we can find the importance of multiple tumor marker detection. For instance, using tumor marker CA19-9 can only detect 60% patients of pancreatic cancer, but when combined with CA125, CA50 and DU-PAN, more than 90% patients can be detected. Using CEA can detect a high percent of patients of lung cancer, but when combined with NSE, Sialic, and SCC, the positive ratio will be higher.

Another advantage of the protein chip for parallel detection of multiple markers is its high sensitivity. Using chemiluminescence and enhanced chemiluminescence, the protein chip has a capacity to detect 1 picogram target protein in 1 ml body fluids. Some markers with high specificity but very low concentration in body fluids can be detected with protein chip technology, which makes more markers optional for clinical detection.

Protein chip provides a high-performed, convenient, accurate defection method for modern medical diagnosis, and it has a very important significance on the early-phase disease diagnosis, timely therapy of difficult diseases and malignant diseases (such as tumors). Because protein is a better index to express living activity than gene, protein chip has more directly application prospects than gene chip on the related fields of disease diagnosis and therapy, development of novel drugs and molecular mechanism exploration of diseases.

In evaluation items of clinical diagnostic kit, the aspects of present protein chip detection system are:
Sensitivity: true positive / (true positive + false negative) >80%;
   The detection limit is 10⁻¹² g/ml when serum sample is used.
Specificity: true positive / (true positive + false negative) >95%.
Stability: protein chips are stable for 2 years when stored at 4□ and for 1 week when stored at 37□.
Convenience: detection with protein chip can be finished in 1.5 hours.
Security: no radioactivity and no pollution.

### BRIEF DESCRIPTION OF DARWINGS

Fig. 1 shows the detection result of standard serum sample with the protein chip
Fig. 2 shows the detection result of serum sample of patient with liver cancer with the protein chip.

### DESCRIPTION OF PREFERRED EMBODIMENTS

The examples followed will illustrate the present invention, but they do not limit in the present invention.

### Example 1, preparation of the protein chip

Solid substrate: nitrocellulose.

Proteins immobilized on solid substrate: the monoclonal antibodies against tumor markers with an amount of 5ng and in a density of 25 dot/0.36cm².

Immobilized proteins: A1, A2―CA153 antibody, A3, A4―CA19-9 antibody, A5― negative control, B1,B2―CA125 antibody, B3,C4―AFP antibody, C1,C2―CA125 antibody, C3,B4―beta-HCG antibody, B5,C5―CA19-5 antibody, D1,D2―PSA antibody, D3,E4―CEA antibody, E1,E2―PSA antibody, E3,D4―CA15-3 antibody, D5,E5―CA549 antibody.

All of the antibodies are purchased from Fitzgerald and CanAg.

AFP―α-Fetoprotein, CEA―Carcinoembryonic antigen, PSA―Prostate specific antigen, betaHCG―Human chorionic gonadotropin beta subunit, CA―Carbohydrate antigen, positive control―no substance.

Coating solution: CBS (NaHCO₃-Na₂CO₃) at pH9.6.

Blocking solution: 0.9%NaCI, 1.21%Tris, 0.2%Tween20, 0.1%tyrosine, 4%sucrose, 5%BSA and 0.5%proclin.

### Preparation steps:

1) dissolving the proteins to be immobilized onto the protein chip with 25% coating solution, then immobilizing these proteins onto the solid substrate by an automatic spotting system (BioGrid Total Array System, BioRobotics Company).
2) leaving overnight.
1) blocking the protein chip with blocking solution; drying and storing at 4□.

### Example 2, protein detection

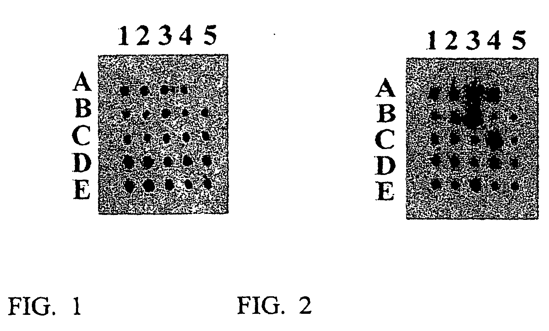

Figure 1 and figure 2 show the result of normal sample serum and liver cancer patient's sample serum being detected by the protein chip.

Said two sample sera are provided by Shanghai Ninth People's Hospital.

The immobilizing positions of proteins are the same to that in example one.

### Operating steps:

1) diluting the supernatant liquid of sample 1:5 with the diluent;
2) sucking the diluted solution and dropping onto the surface of protein chip with a density of 500µl volume per cm²; vibrating at a frequency of 300 r/min and a temperature of 37□ for 25 minutes; the tumor markers in body fluid would react with monoclonal antibody immobilized on the protein chip, and forming stable antigen-antibody complex;
3) removing the solution after the reaction; immersing the protein chip in washing solution and vibrating for 4 minutes; repeating for three times;
1) sucking the mixture of various monoclonal antibodies labeled with peroxidase (purchased from Fitzgerald, CanAg company) and dropping them onto the surface of protein chip with a density of 500µl volume per cm²; vibrating at a frequency of 300 r/min and a temperature of 37□ for 25 minutes, so that the antigen-antibody complex can further combine with another complemantary monoclonal antibody in the mixture of various monoclonal antibodies and to form a stable antibody-antigen-another monoclonal antibody (labeled with HPR) complex;
5) removing the solution after the reaction; immersing the protein chip in washing solution and vibrating for 4 minutes; repeating for three times;
1) drying the chip at room temperature; dropping the chemical composition which can generate chemiluminescence onto chip surface with a density of 100µl volume per cm² ; reacting for 3 minutes at 37□; Chemical composition Luminol (5-amino-2, 3-dihydro-1, 4-phthalazinedione Sodium Salt), H₂O₂, Enhancer (Eosin or PIP) and peroxidase (etc. horseradish peroxidase, HPR), are all purchased from Sigma.
7) taking out the chip and detecting light signals at the 10th minute; imaging the light signals through lens and converting light signals to digital signals; quantitatively analyzing signals by using a software; the minimum signal can be detected is 10⁻¹² g when converted the into protein amount.

As the detection results showed in the figures, the signal at the position of (A3, A4) and (B3, C4) have great difference between normal specimen and liver cancer patient's specimen, which is corresponding to CA19-9 and AFP.

### UTILITY IN INDUSTRY

The advantages and prospects of present invention are very distinct. The protein chip can significantly improve detection accuracy, and has high reliability and sensitivity. The protein chip can be widely used in areas of molecular biology, biomedicine, and human proteomics study, especially in clinical diagnosis.

## Claims

1. A protein chip that comprises a solid substrate and proteins immobilized on solid substrate for parallel detection of multiple markers.

2. The protein chip of claim 1, wherein said proteins can be antigens, antibodies, receptors and ligands.

3. The protein chip of claim 2, wherein said proteins can specifically bind with disease-related proteins.

4. The protein chip of claim 3, wherein said proteins can specifically bind with tumor marker.

5. The protein chip of claim 1, wherein said solid substrate can be inorganic substrate or organic substrate.

6. The protein chip of claim 5, wherein said solid substrate can be cellulose acetate membrane, nitrocellulose membrane, nylon membrane and polypropylene membrane.

7. The protein chip of claim 5, wherein said solid substrate is semiconductor silicon wafer, glass wafer, micro-pore silicon wafer and micro-pore glass.

8. The protein chip of claim 1, wherein the density of said proteins immobilized on the substrate is not lower than 25 dots per cm².

9. The protein chip of claim 1, wherein the amount of said proteins immobilized on the substrate ranges from 0.1 ng/dot to 10ng/dot.

10. A method for preparing protein chip comprising the steps of:
(a) immobilizing various proteins on the substrate in a predetermined order;
(b) blocking the regions without immobilized proteins on the solid substrate with blocking solution; drying and storing.

11. The preparation method of claim 10, wherein said proteins are immobilized on the surface of substrate by covalent linkage or physical absorption.

12. The preparation method of claim 10, wherein said blocking solution comprises sucrose, BSA, Tween20, PBS and tyrosine.

13. The preparation method of claim 12, wherein said blocking solution comprises 0.1-0.5%Tween, 0.02-0.3%tyrosine, 1-9%sucrose, 1-9%BSA and 0.1-1%proclin.

14. The preparation method of claim 12, wherein said blocking solution comprises 0.2%Tween, 0.1%tyrosine. 4%sucrose, 5%BSA and 0.5%proclin.

15. The preparation method of claim 11, wherein said proteins are dissolved in coating solution beforehand, and the coating solution is NaHCO₃-Na₂CO₃ buffer at pH9.0-10.0.

16. A protein chip detection system for parallel detection of multiple markers, using chemiluminescence reaction to detect the amount of multiple biomolecules in body fluids, wherein said system comprising:
(a) a protein chip for parallel detection of multiple markers;
(b) a mixture of various proteins labeled with peroxidase;
(c) a diluent for diluting sample body fluids;
(d) a chemical composition that can generate and enhance chemoluninescence reaction;
(e) a washing solution;
(f) a series of standards of protein to be detected.

17. The detection system of claim 16, wherein said peroxidase is horseradish peroxidase.

18. The detection system of claim 16, wherein said mixture of various proteins is labeled with peroxidase, which can specifically bind with target proteins in body fluids and are mixed at a certain concentration.

19. The detection system of claim 16, wherein said washing solution comprises 0.7-0.9%NaCI, 1.0-1.5%Tris, 0.05-0.2%Tween 20 and pH ranges from 6.0 to 9.0.

20. The detection system of claim 19, wherein said the optimum ingredient of washing solution comprises 0.9%NaCl, 1.21%Tris, 0.1%Tween20 at pH 7.50.

21. The detection system of claim 16, wherein said diluent comprises 0.7-0.9%NaCl, 1.0-1.5%Tris, 0.05-0.2%Tween20 and 0.05-0.2%tyrosine.

22. The detection system of claim 21, wherein said optimum ingredient of diluent comprises 0.9%NaCI, 1.21%Tris, 0.1%Tween20 and 0.1%tyrosine.

23. A detection method by using the protein chip detection system of claim 16 comprising the steps of:
(1)contacting the sample solutions with the solid substrate immobilized with various proteins(B) that can specifically bind with corresponding target proteins(A) in sample solutions, forming stable complex B-A;
(2) complex (B-A) further reacting with the corresponding protein (C-labeled), which can specifically bind with A, to form stable A-B-C-labeled complex; the label is a chemiluminescent marker;
(3) adding chemical composition which can lead to chemiluminescence and generate light signal;
(4) detecting light signals.

24. The detection method of claim 23, wherein said label is horseradish peroxidase.

25. The detection method of claim 23, wherein said mixture of various proteins, labeled with peroxidase, can specifically bind with target proteins in body fluids and are mixed at a certain concentration.

26. The detection method of claim 24 or 25, wherein said peroxidase is horseradish peroxidase.

## Amended claims

### Amended claims under Art. 19.1 PCT

**1.** A protein chip that comprises an organic substrate and proteins immobilized on the substrate, wherein said proteins can specifically bind with multiple tumor markers.

**2.** The protein chip of claim 1, wherein said organic substrate can be cellulose acetate membrane, nitrocellulose membrane, nylon membrane and polypropylene membrane.

**3.** The protein chip of claim 2, wherein the optimum organic substrate is nitrocellulose membrane.

**4.** The protein chip of claim 1, wherein the density of said proteins immobilized on the substrate is not lower than 25 dots per cm².

**5.** The protein chip of claim 1, wherein the amount of said proteins immobilized on the substrate ranges from 0.1 ng/dot to 10ng/dot.

**6.** A method for preparing protein chip comprising the steps of:
(1) dissolving the various proteins to be immobilized with coating solution at certain concentration;
(2) immobilizing various proteins on the organic substrate by an automatic spotting system in a density of 25-200 dot/cm² wherein the amount of the said protein ranges from 0.1 to lung/dot;
(3) leaving for a period of time and blocking the chip with blocking solution; drying and storing at 4°C.

**7.** The preparation method of claim 6, wherein said blocking solution is TBS (0.7-0.9%NaCI, 1.0-1.5%Tris) containing sucrose, BSA, Tween20, tyrosine and proclin.

**8.** The preparation method of claim 7, wherein said blocking solution comprises 0.1-0.5%Tween, 0.02-0.3%tyrosine, 1-9%sucrose, 1-9%BSA and 0.1-1%proclin.

**9.** The preparation method of claim 8, wherein said optimum ingredient of blocking solution is TBS containing 0.2%Tween, 0.1%tyrosine, 4%sucrose, 5%BSA and 0.5%proclin.

**10.** The preparation method of claim 6, wherein said coating solution is NaHCO₃-Na₂CO₃ buffer at pH9.0-10.0.

**11.** The preparation method of claim 6, wherein said organic substrate can be cellulose acetate membrane, nitrocellulose membrane, nylon membrane and polypropylene membrane.

**12.** The preparation method of claim 11, wherein said preferred organic substrate is nitrocellulose membrane.

**13.** The preparation method of claim 6, wherein said the proteins can be antigens, antibodies, receptors and ligands.

**14.** The preparation method of claim 13, wherein said proteins can specifically bind with disease-related proteins.

**15.** The preparation method of claim 14, wherein said proteins can specifically bind with tumor markers.

**16.** A protein chip detection system for parallel detection of multiple markers, using chemiluminescence reaction to detect the amount of multiple biomolecules in body fluids, wherein said system comprising:
(1) a protein chip for parallel detection of multiple markers;
(2) a mixture of various proteins labeled with peroxidase ;
(3) a diluent for diluting sample body fluids;
(4) a chemical composition that can generate and enhance chemoluninescence reaction;
(5) a washing solution;
(6) a series of standards of protein to be detected.

**17.** The detection system of claim 16, wherein said peroxidase is horseradish peroxidase.

**18.** The detection system of claim 16, wherein said mixture of various proteins is labeled with peroxidase, which can specifically bind with target proteins in body fluids and are mixed at.a certain concentration.

**19.** The detection system of claim 16, wherein said washing solution comprises 0.7-0.9%NaCl, 1,0-1,5%Tris, 0.05-0.2%Tween 20 and pH ranges from 6.0 to 9.0.

**20.** The detection system of claim 19, wherein said the optimum ingredient of washing solution comprises 0.9%NaCI, 1.21%Tris, 0.1%Tween20 at pH 7.50.

**21.** The detection system of claim 16, wherein said diluent comprises 0.7-0.9%NaCI, 1.0-1.5%Tris, 0.05-0.2%Tween20 and 0.05-0.2%tyrosine.

**22.** The detection system of claim 21, wherein said optimum ingredient of diluent comprises 0.9%NaCl, 1.21%Tris, 0.1%Tween20 and 0.1%tyrosine.

**23.** A detection method by using the protein chip detection system of claim 16 comprising the steps of:
(1) contacting the sample solutions with the solid substrate immobilized with various proteins(B) that can specifically bind with corresponding target proteins(A) in sample solutions, forming stable complex B-A;
(2)complex (B-A) further reacting with the corresponding protein(C-labeled), which can specifically bind with A, to form stable A-B-C-labeled complex; the label is a chemiluminescent marker;
(3) adding chemical composition which can lead to chemiluminescence reaction and generate light signal;
(4)detecting light signals.

**24.** The detection method of claim 23, wherein said label is horseradish peroxidase.

**25.** The detection method of claim 23, wherein said mixture of various proteins, labeled with peroxidase, can specifically bind with target proteins in body fluids and are mixed at a certain concentration.

**26.** The detection method of claim 24 or 25, wherein said peroxidase is horseradish peroxidase.
